(19) Europäisches Patentamt
European Patent Office
Office européen des brevets

(11) **EP 4 521 356 A1**

(12) **EUROPEAN PATENT APPLICATION**

(43) Date of publication:
**12.03.2025 Bulletin 2025/11**

(21) Application number: **24185099.9**

(22) Date of filing: **27.06.2024**

(51) International Patent Classification (IPC):
**G06V 10/26** (2022.01)   **G06V 10/44** (2022.01)

(52) Cooperative Patent Classification (CPC):
**G06V 10/26; G06V 10/44;** G06V 2201/031

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.09.2023 KR 20230117642**

(71) Applicant: **Medicalip Co., Ltd.**
**Gangwon-do 24341 (KR)**

(72) Inventors:
• **PARK, Sang Joon**
**Seoul (KR)**
• **KIM, Jong Min**
**Yongin-si, Gyeonggi-do (KR)**
• **CHO, Dong Jin**
**Seoul (KR)**

(74) Representative: **Prendin, Marco et al**
**Cantaluppi & Partners S.r.l.**
**Piazzetta Cappellato Pedrocchi, 18**
**35122 Padova (IT)**

(54) **METHOD AND APPARATUS FOR DETECTING DIAMETER OF TUBULAR STRUCTURE IN MEDICAL IMAGE**

(57)    Provided are a method and an apparatus for detecting the diameter of a tubular structure in a medical image. The apparatus for detecting the diameter of a tubular structure is configured to identify the tubular structure in a two-dimensional or three-dimensional medical image, calculate a distance map indicating a distance from each point inside the tubular structure to a boundary of the tubular structure, calculate central points, based on a gradient of a distance value in the distance map, connect the central points to create a center line, and obtain the diameter of the tubular structure for each location by determining a distance between the center line and the boundary of the tubular structure in a direction perpendicular to the center line.

FIG. 1

## Description

This application is based on the support project of the Ministry of Health and Welfare (Task number: 1465034178, Task number: HI21C1074050021, Research project name: Construction of big data specialized in intensive care and developing AI-based CDSS).

CROSS-REFERENCE TO RELATED APPLICATION

This application is based on and claims priority under 35 U.S.C. §119 to Korean Patent Application No. 10-2023-0117642, filed on September 05, 2023, in the Korean Intellectual Property Office, the disclosure of which is incorporated by reference herein in its entirety.

BACKGROUND

1. Field

The disclosure relates to a method and apparatus for detecting the diameters of tubular structures in medical images, and more particularly, to a method and apparatus for detecting the diameters of tubular structures such as aorta for diagnosis of lesions.

2. Description of the Related Art

The shapes of tubular structures such as arteries may be identified from medical images. For example, lesions may be diagnosed by identifying the shapes of arteries and the like from a completed tomography (CT) or magnetic resistance imaging (MRI) image. Although an aorta aneurysm occurs slowly without major symptoms, continuous monitoring and early detection are important for diseases such as aorta aneurysms as the mortality rate is more than 80 % after an acute rupture occurs. Although medical staff may directly measure the diameters of blood vessels in medical images, it is difficult to accurately measure the diameter change of a gradually progressing aorta aneurysm without error every time, and it takes much time because the diameters should be obtained from various locations of blood vessels.

SUMMARY

The disclosure provides a method and an apparatus for automatically detecting a diameter of a tubular structure in a medical image.

Additional aspects will be set forth in part in the description which follows and, in part, will be apparent from the description, or may be learned by practice of the presented embodiments of the disclosure.

According to an aspect of the disclosure, a method of detecting a diameter of a tubular structure includes identifying the tubular structure in a two-dimensional or three-dimensional medical image, obtaining a distance map indicating a distance from each point inside the tubular structure to a boundary of the tubular structure, obtaining central points based on a gradient of a distance value in the distance map, creating a center line by connecting the central points, and obtaining the diameter of the tubular structure for each location by determining a distance between the center line and the boundary of the tubular structure in a direction perpendicular to the center line.

According to another aspect of the disclosure, an apparatus for detecting a diameter of a tubular structure includes a structural identification unit configured to identify the tubular structure in a two-dimensional or three-dimensional medical image, a distance calculation unit configured to calculate a distance map indicating a distance from each point inside the tubular structure to a boundary of the tubular structure, a center calculation unit configured to calculate central points based on a gradient of a distance value in the distance map and connect the central points to create a center line, and a diameter detection unit configured to determine a distance to the boundary of the tubular structure in a direction perpendicular to the center line and detect the diameter of the tubular structure for each location.

BRIEF DESCRIPTION OF THE DRAWINGS

The above and other aspects, features, and advantages of certain embodiments of the disclosure will be more apparent from the following description taken in conjunction with the accompanying drawings, in which:

FIG. 1 is a diagram illustrating an example of a diameter detection apparatus for detecting the diameter of a tubular structure, according to an embodiment;

FIGS. 2 and 3 are flowcharts illustrating an example of a method of detecting the diameter of a tubular structure, according to an embodiment;

FIG. 4 is a diagram illustrating an example of a method of segmenting a tubular structure from a medical image by using an artificial intelligence model, according to an embodiment;

FIG. 5 is a diagram illustrating an example of detecting the diameter of a tubular structure from a two-dimensional (2D) medical image by using a diameter detection method, according to an embodiment;

FIG. 6 is a diagram illustrating an example of detecting the diameter of a tubular structure from a three-dimensional (3D) medical image by using a diameter detection method, according to an embodiment; and

FIG. 7 is a diagram illustrating a configuration of an example of a diameter detection apparatus according to an embodiment.

DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, a method and an apparatus for detecting the diameter of a tubular structure according to an embodiment of the present invention will be described below in detail with reference to the accompanying drawings.

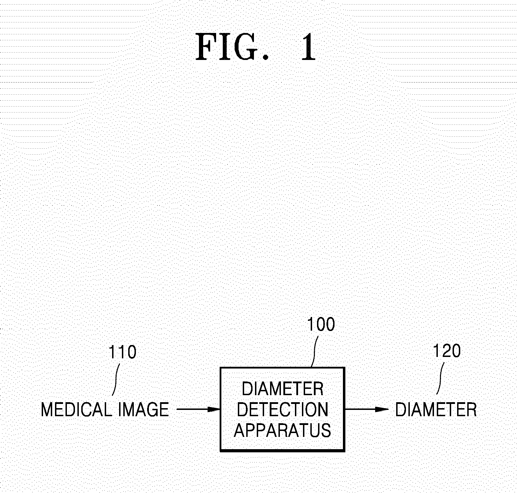

FIG. 1 is a diagram illustrating an example of a diameter detection apparatus of detecting diameter of a tubular structure according to an embodiment.

Referring to FIG. 1, when receiving a medical image 110 as an input, a diameter detection apparatus 100 identifies a tubular structure region in the medical image and then detects the diameters 120 of the tubular structure at various locations. Here, the medical image 110 may be a 2D medical image such as an X-ray image or a 3D medical image such as CT or MRI.

The tubular structure in the medical image 110 is a narrow and long tubular structure, and may be various types of human tissues such as arteries (e.g., aorta), veins, and airways. However, hereinafter, for convenience of explanation, an example of detecting the diameter of the aorta will be mainly described below.

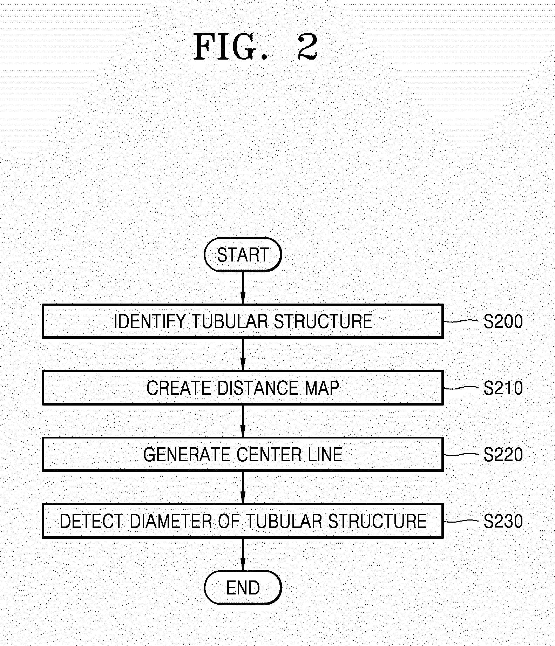

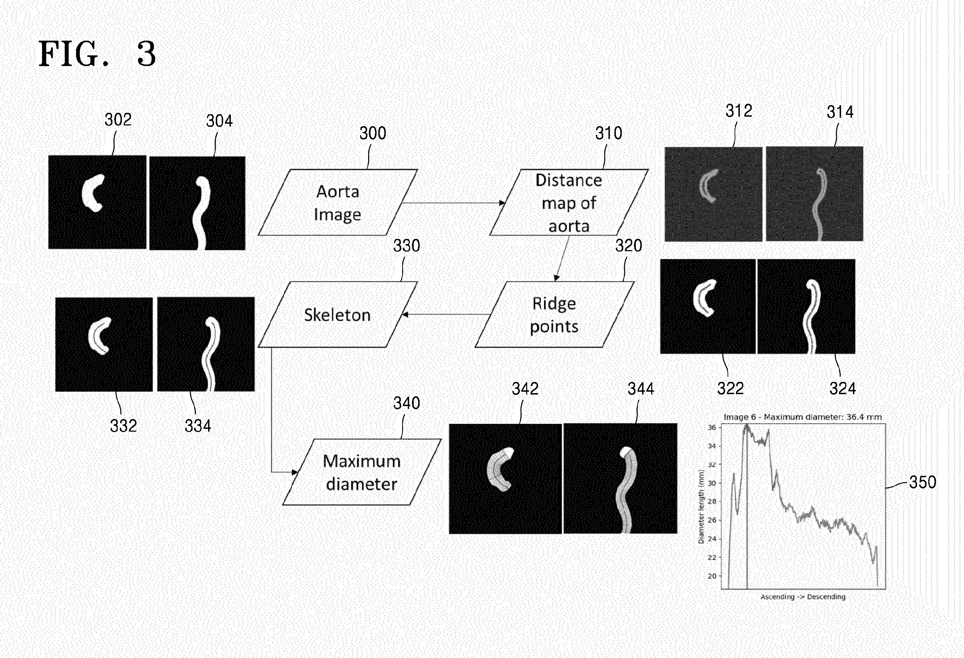

FIGS. 2 and 3 are flowcharts illustrating an example of a method of detecting the diameter of a tubular structure according to an embodiment.

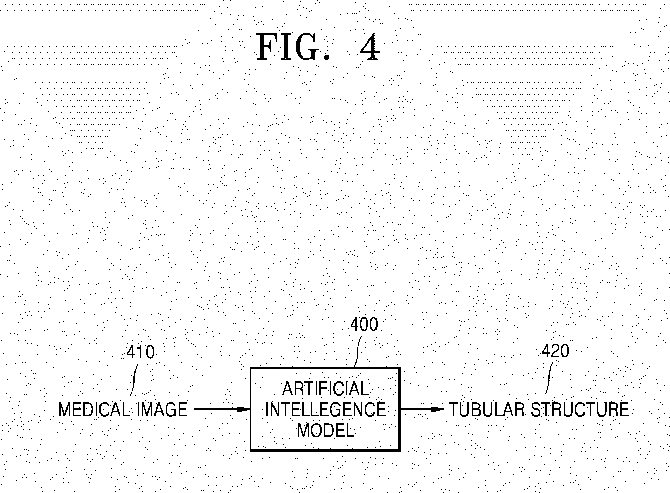

Referring to FIGS. 2 and 3, the diameter detection apparatus 100 identifies at least one tubular structure (e.g., the aortic image 300) in a medical image 110 (S200). For example, the diameter detection apparatus 100 may segment an aortic region from a 2D or 3D medical image capturing the chest. Various types of algorithms for segmenting a specific human tissue in the medical image 110 may be applied to the present embodiment. Alternatively, the diameter detection apparatus 100 may use an artificial intelligence model to segment at least one tubular structure in the medical image 110, which will be described again in FIG. 4.

In another embodiment, the diameter detection apparatus 100 may convert the medical image 110 into a binary image including a region of the tubular structure. For example, the diameter detection apparatus 100 may generate a binary image in which the values of the coordinates in the tubular structure region are converted to "0" (or white), and the values of the coordinates in the background region outside the tubular structure region are converted to "1" (or black). FIG. 3 illustrates an example of obtaining a diameter based on binary images 302 and 304 of the aorta. The value of each coordinate in the binary image may be expressed by the following function.

【Equation 1】

$$f(x,y) = \begin{cases} 1 \ (tubular \ structure) \\ 0 \ (background) \end{cases}$$

Here, (x,y) denotes coordinates in the medical image. Equation 1 is an example of a 2D medical image, and in the case of a 3D medical image, the coordinates may include (x, y, z).

The diameter detection apparatus 100 generates a distance map 310 indicating a distance from each point inside the tubular structure to a boundary of the tubular structure (S210). The diameter detection apparatus 100 may obtain a distance map using Euclidean distance transform. For example, the diameter detection apparatus 100 may identify, as a distance value at any one point, the shortest distance from the point in the tubular structure (i.e., pixel coordinates of a 2D medical image and voxel coordinates of a 3D medical image) to points that form the boundary of the tubular structure. For example, the distance map may be a map showing a distance value of each point (coordinate or voxel) in the tubular structure. the distance maps 312 and 314 may be expressed in different colors according to distance values. The distance value d(x,y) at a specific point (x,y) of the 2D medical image may be expressed as the following equation.

【Equation 2】

$$d(x,y) = \min_{f(x',y')=0} \sqrt{(x-x')^2 + (y-y')^2}$$

Here, (x,y) means a coordinate value of a specific point in the tubular structure, and (x',y') means a coordinate value of a boundary point of the tubular structure.

The diameter detection apparatus 100 obtains central points (i.e., ridge points 320) based on the gradient of the distance value in the distance map 310, and connects the central points to generate a center line 330 (i.e., skeleton) (S220). In an embodiment, the diameter detection apparatus 100 may determine, as the central points 320, points in the distance map 310 where the magnitude of the gradient of the distance value is less than or equal to a preset threshold. This is expressed in a mathematical equation as follows.

【Equation 3】

$$(x,y) \ such \ that \ \sqrt{\left(\frac{\partial d}{\partial x}\right)^2 + \left(\frac{\partial d}{\partial y}\right)^2} \leq threshold$$

Here, d denotes a distance value obtained in Equation 2, and the threshold may be predefined as various values according to embodiments. A coordinate value of (x, y) satisfying Equation 3 becomes the central points 320.

In another embodiment, the central points 320 may be spaced apart by a predetermined distance or noise may exist therein. In this case, the diameter detection apparatus may add central points using interpolation between the central points 320 spaced apart from each other by a predetermined distance, or connect and smooth the central points. For example, the diameter detection apparatus 100 may connect a plurality of central points 320 using a linear one-dimensional (1D)-interpolation method and smooth the plurality of central points 320 using a 1D-Gaussian filter. In addition, various conventional methods of connecting and smoothing a plurality of central points 320 to make one line segment may be applied to the present embodiment.

The diameter detection apparatus 100 detects the diameter (e.g., the maximum diameter 340) of the tubular structure by using the distance between the center line 330 and the boundary of the tubular structure in a direction perpendicular to the center line 330 (S230). For example, the diameter detection apparatus 100 may obtain a tangent line at each point of the center line, and obtain the distance between the center line and the boundary of the tubular structure using a line segment perpendicular to the tangent line (i.e., a line segment perpendicular to the center line) based on the binary images 342 and 344. The diameter detection apparatus 100 may detect the diameter of the tubular structure at various points of the center line (e.g., central points at regular intervals) or at all points constituting the center line (i.e., all central points). In addition, the diameter detection apparatus 100 may detect and output a maximum value among diameters for respective locations of the tubular structure (as shown in a graph 350 of FIG. 3).

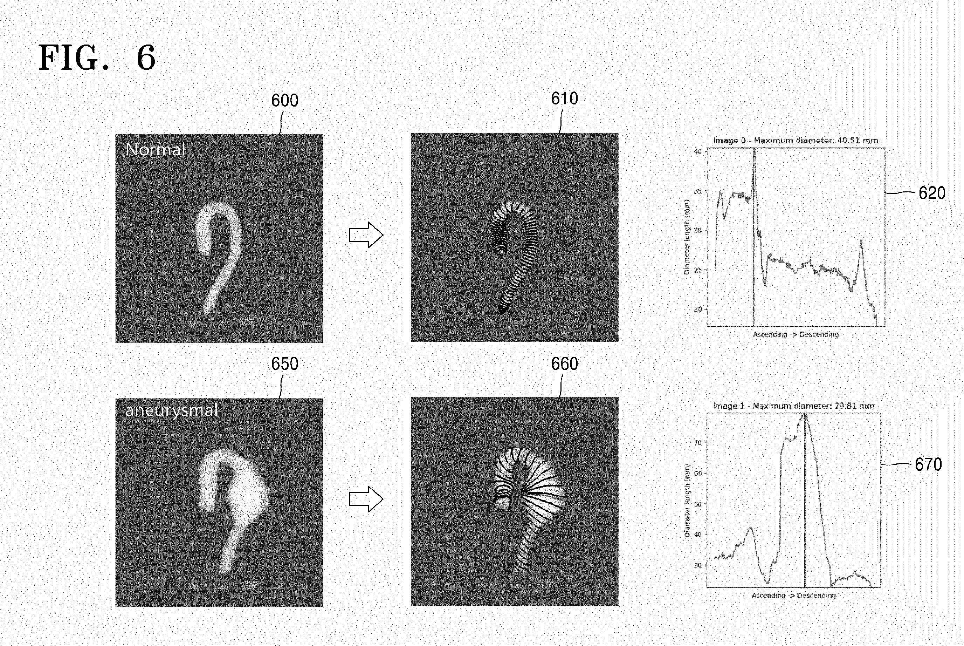

In another embodiment, the diameter detection apparatus 100 may determine a lesion based on the diameter of the tubular structure. For example, the diameter detection apparatus 100 may determine the risk of aneurysm by detecting the diameter of the aorta from a chest medical image. This will be described again with reference to FIG. 6.

FIG. 4 is a diagram illustrating an example of a method of segmenting a tubular structure from a medical image using an artificial intelligence model according to an embodiment.

Referring to FIG. 4, an artificial intelligence model 400 is a model trained to segment the tubular structure 420 from the medical image when receiving the medical image 410 as an input. The artificial intelligence model 400 may be implemented with various types of artificial neural networks such as a convolutional neural network (CNN).

In an embodiment, the artificial intelligence model 400 may be generated by training with a supervised learning method based on training data including medical images labeling the aorta. Since the training process of the artificial intelligence model 400 itself is already a widely known method, an additional explanation thereof will be omitted.

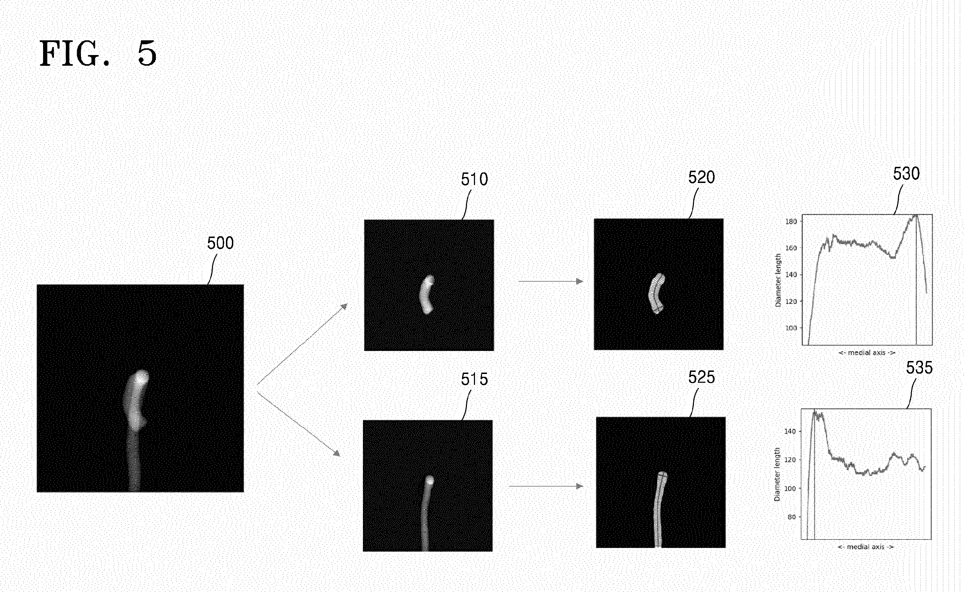

FIG. 5 is a diagram illustrating an example of detecting the diameter of a tubular structure from a two-dimensional (2D) medical image using a diameter detection method according to an embodiment.

Referring to FIG. 5, the diameter detection apparatus 100 acquires an image 500 obtained by segmenting a tubular structure from a 2D medical image. Since the 2D medical image is displayed on one plane by overlapping a plurality of tissues, the tubular structure may be overlapped and displayed on an image 500 obtained by segmenting a region of the tubular structure from the medical image. For example, the aorta may appear to overlap some sections as shown in the image 500 of FIG. 5 according to the photographing direction.

The diameter detection apparatus 100 segments each tubular structure from the image 500 when the tubular structures overlap. In an embodiment, the diameter detection apparatus 100 may segment overlapping tubular structures using the brightness value within the image 500. Various existing image analysis algorithms or artificial intelligence models for dividing overlapped images may be applied to the present embodiment.

The diameter detection apparatus 100 performs diameter detection from the images 520 and 525 on the tubular structures 510 and 515. For example, the diameter detection apparatus 100 determines the center line on each tubular structure, as seen in FIGS. 2 and 3, and detects the diameters of the tubular structure at various locations based on the center line. The diameter detection apparatus 100 may display a diameter for each position of each tubular structure as shown in graphs 530 and 535.

FIG. 6 is a diagram illustrating an example of detecting the diameter of a tubular structure from a three-dimensional (3D) medical image using a diameter detection method according to an embodiment.

Referring to FIG. 6, the diameter detection apparatus 100 may generate images 600 and 650 each including a tubular structure region based on a brightness (i.e., a Hounsfield unit (HU) value) of a voxel from a 3D medical image. This embodiment presents an example 610 of detecting the aorta diameter in a normal case and an example 660 of detecting the aorta diameter in the presence of an aneurysm. The diameter detection apparatus 100 may output diameters for respective positions to the graphs 620 and 670.

The diameter detection apparatus 100 may determine the risk of aneurysm based on the diameter data of the aorta in a normal case. For example, when the maximum diameter of the aorta is swollen more than a certain amount compared to the normal case, such aorta diameters may be determined as an aneurysm. The diameter detection apparatus 100 may digitize and present the risk of aneurysm based on the difference between the maximum diameter of the normal aorta and the maximum diameter of the swollen aorta.

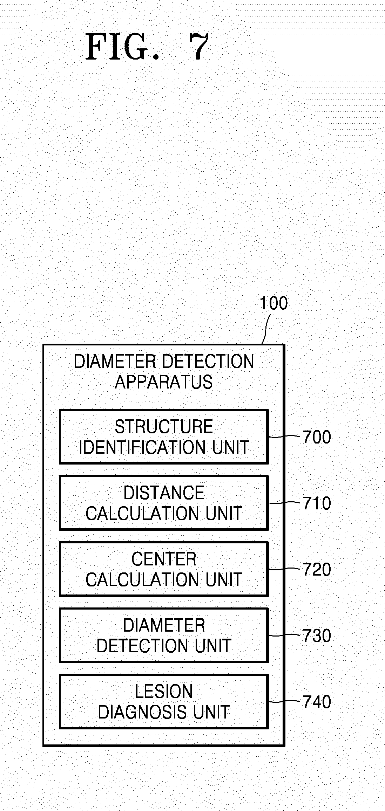

FIG. 7 is a diagram illustrating a configuration of an example of a diameter detection apparatus according to an embodiment.

Referring to FIG. 7, the diameter detection apparatus 100 includes a structure identification unit 700, a distance calculation unit 710, a center calculation unit 720, a diameter detection unit 730, and a lesion diagnosis unit 740. In an embodiment, the lesion diagnosis unit 740 may be omitted. In another embodiment, the diameter detection apparatus 100 may be implemented as a computing device including a memory, a processor, and an input/output device. In this case, each component may be implemented in software (program) and then performed by a processor after being loaded on a memory.

The structure identification unit 700 identifies a tubular structure from a 2D or 3D medical image. For example, the structure identification unit 700 may segment a tubular structure such as the aorta based on a brightness value of the medical image, or segment a tubular structure using the artificial intelligence model of FIG. 4. In another embodiment, the structure identification unit 700 may generate an image including a tubular structure as a binary image.

The distance calculation unit 710 obtains a distance map indicating a distance from each point inside the tubular structure to a boundary of the tubular structure. For example, the distance calculation unit 710 may obtain a distance map indicating the distance to each point in the tubular structure using Euclidean distance transform.

The center calculation unit 720 obtains central points based on a gradient of a distance value in the distance map and connects the central points to create a center line. A kind of ridge may be obtained by connecting points having a small change in distance value at each point in the tubular structure. In addition, various methods for obtaining central points based on a distance map may be applied to the present embodiment and embodiments are not limited to specific examples. In another embodiment, when the central points are not continuous or have an even line shape, the center calculation unit 720 may obtain a center line smoothly connected in a single line by applying interpolation or smoothing.

The diameter detection unit 730 determines the distance to the boundary of the tubular structure in a direction perpendicular to the center line and obtains the diameter of the tubular structure for each position. The diameter detection unit 730 may obtain the diameter of the tubular structure at regular intervals along the center line, or may obtain the diameter of the tubular structure at each and every point located on the center line.

The lesion diagnosis unit 740 determines a lesion based on the diameter of the tubular structure.

For example, the lesion diagnosis unit 740 may compare the diameter of the aorta identified from the medical image with the diameter of the aorta of a normal group stored in advance to determine the occurrence of aneurysm or a degree of risk (a predefined degree of risk according to a difference in diameter size between the normal group and a control group). In another embodiment, the lesion diagnosis unit 740 may determine whether an aneurysm occurs or a degree of risk based on a change in the diameters of the aorta in medical images captured at various times for the same patient. That is, the lesion diagnosis unit 740 may determine that there is a risk of an aneurysm if the maximum diameter of the aorta changes to a predetermined size or more in a plurality of medical images taken at multiple points in time. The lesion diagnosis unit 740 may digitize and output the risk of aneurysm based on a change in the diameter size of the aorta over time.

The present method according to the disclosure may also be implemented as computer-readable program code on a computer-readable recording medium. The computer-readable recording medium includes all types of recording devices in which data readable by a computer system is stored. Examples of computer-readable recording media include ROM, RAM, CD-ROM, magnetic tape, floppy disk, optical data storage device, and the like. In addition, computer-readable recording media are distributed in a network-connected computer system so that computer-readable code may be stored and executed in a distributed manner.

According to an embodiment of the disclosure, the diameter of a tubular structure such as an artery may be automatically determined from a 2D or 3D medical image. In another embodiment, the risk of aneurysm may be numerically provided by detecting the diameter of the aorta.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

The invention could be inter alia defined by the following examples:

1. A method of detecting a diameter of a tubular structure, the method comprising: preferably receiving a two-dimensional (2D) medical image or a three-dimensional (3D), identifying the tubular structure in the two-dimensional (2D) or three-dimensional (3D) medical image;

obtaining a distance map indicating a distance from each point inside the tubular structure to a boundary of the tubular structure;

obtaining central points, based on a gradient of a distance value in the distance map;

creating a center line by connecting the central points; and

obtaining the diameter of the tubular structure for each location by determining a distance between the center line and the boundary of the tubular structure in a direction perpendicular to the center line.

2. The method of example 1, wherein the 2D or 3D medical image is an X-ray image or a CT or MRI image.

3. The method of any one of the preceding examples, wherein the tubular structure comprises an aorta.

4. The method of example 3, further comprising determining a risk of aneurysm, based on a diameter of the aorta.

5. The method of any one of the preceding examples, wherein the identifying of the tubular structure comprises generating a binary image including a region corresponding to the tubular structure in the 2D or 3D medical image.

6. The method of any one of the preceding examples, wherein the obtaining of the distance map comprises generating a distance map including a shortest distance from each point in the tubular structure to a boundary of the tubular structure.

7. The method of any one of the preceding examples, wherein the creating of the center line comprises:

if there are central points spaced apart from each other by a certain distance, adding central points using interpolation; and

connecting and smoothing the central points.

8. An apparatus for detecting a diameter of a tubular structure, the apparatus comprising:

a structural identification unit configured to identify the tubular structure in a two-dimensional or three-dimensional medical image;

a distance calculation unit configured to calculate a distance map indicating a distance from each point inside the tubular structure to a boundary of the tubular structure;

a center calculation unit configured to calculate central points, based on a gradient of a distance value in the distance map, and connect the central points to create a center line; and

a diameter detection unit configured to determine a distance to the boundary of the tubular structure in a direction perpendicular to the center line and detect the diameter of the tubular structure for each location.

9. The apparatus of example 8, further comprising a lesion diagnosis unit configured to, when the tubular structure is an aorta, determine a risk of aneurysm, based on the diameter of the aorta.

10. A computer-readable recording medium on which a computer program for performing the method of any one of the examples 1 to 7 is recorded.

**Claims**

1. A method of detecting a diameter of a tubular structure, the method comprising:

identifying the tubular structure in a two-dimensional (2D) or three-dimensional (3D) medical image;

obtaining a distance map indicating a distance from each point inside the tubular structure to a boundary of the tubular structure;

obtaining central points, based on a gradient of a distance value in the distance map;

creating a center line by connecting the central points; and

obtaining the diameter of the tubular structure for each location by determining a distance between the center line and the boundary of the tubular structure in a direction perpendicular to the center line.

2. The method of claim 1, wherein the 2D or 3D medical image is an X-ray image or a CT or MRI image.

3. The method of claim 1, wherein the tubular structure comprises an aorta.

4. The method of claim 3, further comprising determining a risk of aneurysm, based on a diameter of the aorta.

5. The method of claim 1, wherein the identifying of the tubular structure comprises generating a binary image including a region corresponding to the tubular structure in the 2D or 3D medical image.

6. The method of claim 1, wherein the obtaining of the distance map comprises generating a distance map including a shortest distance from each point in the tubular structure to a boundary of the tubular structure.

7. The method of claim 1, wherein the creating of the center line comprises:

if there are central points spaced apart from each other by a certain distance, adding central points using interpolation; and

connecting and smoothing the central points.

8. An apparatus for detecting a diameter of a tubular structure, the apparatus comprising:

   a structural identification unit configured to identify the tubular structure in a two-dimensional or three-dimensional medical image;
   a distance calculation unit configured to calculate a distance map indicating a distance from each point inside the tubular structure to a boundary of the tubular structure;
   a center calculation unit configured to calculate central points, based on a gradient of a distance value in the distance map, and connect the central points to create a center line; and
   a diameter detection unit configured to determine a distance to the boundary of the tubular structure in a direction perpendicular to the center line and detect the diameter of the tubular structure for each location.

9. The apparatus of claim 8, further comprising a lesion diagnosis unit configured to, when the tubular structure is an aorta, determine a risk of aneurysm, based on the diameter of the aorta.

10. A computer-readable recording medium on which a computer program for performing the method of claim 1 is recorded.

# FIG. 1

110

MEDICAL IMAGE → DIAMETER DETECTION APPARATUS → DIAMETER

100

120

# FIG. 2

START

| IDENTIFY TUBULAR STRUCTURE | — S200 |

| CREATE DISTANCE MAP | — S210 |

| GENERATE CENTER LINE | — S220 |

| DETECT DIAMETER OF TUBULAR STRUCTURE | — S230 |

END

# FIG. 3

*EP 4 521 356 A1*

# FIG. 4

MEDICAL IMAGE ——→ ARTIFICIAL INTELLEGENCE MODEL →— TUBULAR STRUCTURE

410

400

420

FIG. 5

EP 4 521 356 A1

FIG. 6

EP 4 521 356 A1

# FIG. 7

100

| DIAMETER DETECTION APPARATUS |
| --- |
| STRUCTURE IDENTIFICATION UNIT — 700 |
| DISTANCE CALCULATION UNIT — 710 |
| CENTER CALCULATION UNIT — 720 |
| DIAMETER DETECTION UNIT — 730 |
| LESION DIAGNOSIS UNIT — 740 |

**EUROPEAN SEARCH REPORT**

Application Number

EP 24 18 5099

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/033302 A1 (GRADY LEO [US] ET AL) 7 February 2008 (2008-02-07) * abstract * * paragraphs [0003], [0006], [0010], [0011] * * paragraphs [0032] - [0038] * * paragraphs [0055] - [0059] * * figures 1-3 * | 1-10 | INV. G06V10/26 G06V10/44 |
| A | BOUIX S ET AL: "Flux driven automatic centerline extraction", MEDICAL IMAGE ANALYSIS, OXFORD UNIVERSITY PRESS, OXOFRD, GB, vol. 9, no. 3, 1 June 2005 (2005-06-01), pages 209-221, XP027847051, ISSN: 1361-8415 [retrieved on 2005-06-01] * section 5 * | 1-10 | |
| A | CN 115 937 239 A (SHENYANG NEUSOFT INTELLIGENT MEDICAL TECH RESEARCH INSTITUTE CO LTD) 7 April 2023 (2023-04-07) * abstract * * page 1, paragraph 2 * * page 2, paragraph 6 * * figures 3,4 * | 1-10 | **TECHNICAL FIELDS SEARCHED (IPC)** G06V |
| T | Brewster Dc ET AL: "Abdominal Aortic Aneurysm Size and Rupture Risk*", , 1 January 2003 (2003-01-01), XP093229768, MSD Manual Professional Version Retrieved from the Internet: URL:https://www.msdmanuals.com/professiona l/multimedia/table/abdominal-aortic-aneury sm-size-and-rupture-risk [retrieved on 2024-12-02] * the whole document * | | |

-/--

The present search report has been drawn up for all claims

| Place of search | Date of completion of the search | Examiner |
|---|---|---|
| The Hague | 2 December 2024 | Philips, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................
& : member of the same patent family, corresponding document

EPO FORM 1503 03.82 (P04C01)

Europäisches
Patentamt
European
Patent Office
Office européen
des brevets

**EUROPEAN SEARCH REPORT**

Application Number

EP 24 18 5099

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | & Anonymous: "MSD Manuals Global Medical Knowledge", , 2 December 2024 (2024-12-02), XP093229759, Retrieved from the Internet: URL:https://www.msdmanuals.com/ [retrieved on 2024-12-02] * the whole document * ----- | 1-10 | |
| | | | **TECHNICAL FIELDS SEARCHED (IPC)** |

The present search report has been drawn up for all claims

| Place of search | Date of completion of the search | Examiner |
|---|---|---|
| The Hague | 2 December 2024 | Philips, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 5099

This annex lists the patent family members relating to the patent documents cited in the above-mentioned European search report.
The members are as contained in the European Patent Office EDP file on
The European Patent Office is in no way liable for these particulars which are merely given for the purpose of information.

02-12-2024

| Patent document cited in search report | Publication date | Patent family member(s) | Publication date |
|---|---|---|---|
| US 2008033302 A1 | 07-02-2008 | DE 102007018750 A1<br>JP 2007289704 A<br>US 2008033302 A1 | 06-12-2007<br>08-11-2007<br>07-02-2008 |
| CN 115937239 A | 07-04-2023 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230117642